(19) Europäisches Patentamt
European Patent Office
Office européen des brevets

(11) EP 3 888 649 B1

(12) EUROPEAN PATENT SPECIFICATION

(45) Date of publication and mention of the grant of the patent:
**09.08.2023 Bulletin 2023/32**

(21) Application number: **19911372.1**

(22) Date of filing: **24.01.2019**

(51) International Patent Classification (IPC):
***A61K 31/352*** *(2006.01)* ***A61P 17/02*** *(2006.01)*
***A61K 45/06*** *(2006.01)*

(52) Cooperative Patent Classification (CPC):
(C-Sets available)
**A61K 45/06; A61K 31/136; A61K 31/352; A61K 31/635; A61K 31/7048; A61K 33/38; A61K 36/185; A61P 17/02** (Cont.)

(86) International application number:
**PCT/CN2019/072916**

(87) International publication number:
**WO 2020/150949 (30.07.2020 Gazette 2020/31)**

(54) **CANNFLAVIN A FOR USE IN PROMOTING WOUND HEALING AND TREATING OR PREVENTING DIABETIC FEET**

CANNFLAVIN A ZUR VERWENDUNG BEI DER FÖRDERUNG DER WUNDHEILUNG UND BEHANDLUNG ODER VORBEUGUNG VON DIABETISCHEN FÜSSEN

CANNFLAVINE A POUR UNE UTILISATION DANS LA PROMOTION DE LA CICATRISATION DES PLAIES ET LE TRAITEMENT OU LA PRÉVENTION DES PIEDS DIABÉTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.10.2021 Bulletin 2021/40**

(73) Proprietor: **Hanyi Bio-Technology Company Ltd.**
**Beijing 100020 (CN)**

(72) Inventors:
- **LI, Xiangdong**
  **Beijing 100020 (CN)**
- **ZHANG, Ke**
  **Beijing 100020 (CN)**
- **TAN, Xin**
  **Beijing 100020 (CN)**
- **YU, Zhaohui**
  **Beijing 100020 (CN)**
- **CHANG, Tanran**
  **Beijing 100020 (CN)**
- **LIAN, Meng**
  **Beijing 100020 (CN)**

(74) Representative: **Murgitroyd & Company**
**Murgitroyd House**
**165-169 Scotland Street**
**Glasgow G5 8PL (GB)**

(56) References cited:
**WO-A1-2006/017892 CN-A- 107 072 979**

- **AHMED M ET AL: "PHYTOCHEMICAL SCREENING, TOTAL PHENOLIC AND FLAVONOIDS CONTENTS AND ANTIOXIDANT ACTIVITIES OF CITRULLUS COLOCYNTHIS L. AND CANNABIS SATIVA L.", APPLIED ECOLOGY AND ENVIRONMENTAL RESEARCH, vol. 17, no. 3, 8 October 2019 (2019-10-08), pages 1-19, XP055862116, ISSN: 1589-1623, DOI: 10.15666/aeer/1703_69616979 Retrieved from the Internet: URL:https://www.epa.hu/02500/02583/00059/pdf/EPA02583_applied_ecology_2019_3_69616979.pdf>**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ROBLEDO SM ET AL: "Studies In Vitro and In Vivo of antileishmanial activity and Differential Cytotoxicity of Cannabis spp.", 65TH INTERNATIONAL CONGRESS AND ANNUAL MEETING OF THE SOCIETY FOR MEDICINAL PLANT AND NATURAL PRODUCT RESEARCH (GA 2017), vol. 4, 1 October 2017 (2017-10-01), XP055862142, ISSN: 2509-6656, DOI: 10.1055/s-0037-1608170 Retrieved from the Internet: URL:http://dx.doi.org/10.1055/s-0037-16081 70>**
- **FRANKOVA A ET AL: "In vitro Antimicrobial and Antioxidant Activity of Extracts from Six Chemotypes of Medicinal Cannabis", PLANTA MEDICA, vol. 81, no. S 01, 14 December 2016 (2016-12-14), pages S1-S381, XP055862162, DE ISSN: 0032-0943, DOI: 10.1055/s-0036-1596302**
- **BARRETT ML ET AL: "Cannflavin A and B, prenylated flavones from Cannabis sativa L", EXPERIENTIA, SPRINGER BASEL AG, CH, vol. 42, no. 4, 15 April 1986 (1986-04-15) , page 452/453, XP002114248, ISSN: 0014-4754, DOI: 10.1007/BF02118655**
- **JOHN M MCPARTLAND: "Cannabis as repellent and pesticide", INTERNET CITATION, 2 December 1997 (1997-12-02), pages 1-8, XP009525640, Retrieved from the Internet: URL:https://www.druglibrary.org/olsen/hemp /iha/jiha4210.html [retrieved on 2019-09-26]**


(52) Cooperative Patent Classification (CPC): (Cont.)

C-Sets
**A61K 31/136, A61K 2300/00;**
**A61K 31/352, A61K 2300/00;**
**A61K 31/635, A61K 2300/00;**
**A61K 31/7048, A61K 2300/00;**
**A61K 33/38, A61K 2300/00**

## Description

### TECHNICAL FIELD

The present invention belongs to the field of medicine, and relates to cannflavin A for use in promoting wound healing. Particularly, the present invention relates to Cannflavin A for use in promoting wound healing of type II diabetes.

### BACKGROUND

Diabetes is a metabolic disease with hyperglycemia as its obvious symptom, which is often divided into type I, type II and gestational diabetes, etc.[1] The type I diabetes, formerly known as insulin-dependent diabetes[2], has a relatively higher incidence in children and adolescents, and can also occur at various ages. The onset of the disease is relatively sharp, and insulin in the body is absolutely insufficient, so ketoacidosis is easy to occur, which must be treated with insulin, otherwise it will endanger life. The type II diabetes, formerly known as adult-onset diabetes[3], usually occurs after 35-40 years old, and accounts for 90% or above of diabetic patients. The ability of producing insulin in patients with type II diabetes is not completely lost, and some patients even produce too much insulin *in vivo*, but the effect of insulin is poor. That is, insulin resistance occurs.

Diabetes is often accompanied by complications, such as diabetic nephropathy, diabetic cardiomyopathy, diabetic peripheral neuropathy, and the like. A wound caused by diabetes is not easy to heal, which really puzzles the diabetic patients and seriously affects their normal life. The wound caused by diabetes[4] can be generally divided into two categories, a first category is a non-ulcerative wound, and a second category is an ulcerative wound, such as diabetic feet. These two kinds of wounds are not easy to heal. The diabetes patients suffer from a series of endocrine and metabolic disorders because they are in a state of persistent hyperglycemia for a long time, which leads to the decline of immunity. In addition, the existence of vasculopathy and neuropathy in lower limbs leads to the anaesthesia or insensitivity of feet, which makes the feet of the diabetes patients more vulnerable to trauma. Skin wounds of general healthy people generally recover substantially in 3-7 days, and most of them only need simple disinfection and dressing. Wounds of burns and scalds need some ointments for drawing out pus and toxin and eliminating necrotic tissues and promoting granulation to accelerate wound healing. However, because the wounds of the diabetic patients need fine debridement and medication which are different from those of ordinary people, infection caused by improper treatment often occurs, which makes the wounds relapse and more difficult to heal, and currently there have been no specific medicine.

Currently, in addition to lowering blood glucose, debridement and preventing infection, the strategies for treating diabetic feet mostly adopt the method of direct and external application of a drug, such as an ointment for invigorating the circulation of blood and relaxing sinew, a rotten foot ointment and some burn and scald drugs, to the affected area, but the drug only slightly relieves symptoms and cannot cure the disease. Many infections such as gram-positive cocci, negative bacilli, anaerobic bacteria, and the like, are often easy to co-exist at a site of skin ulceration, so broad-spectrum antibiotics assisted by metronidazole are selected for fighting against anaerobic bacteria. The application time is long, and long-term use of antibiotics will cause drug resistance or drug tolerance. For patients with diabetic feet or skin ulceration, what is needed very much is not antibiotics, but specific drugs that have super-strong effects of drawing out pus and toxin and eliminating necrotic tissues and promoting granulation, and can improve the internal environment of the affected area to provide a good environment for the regrowth of the affected area. All the time, people at home and abroad have never stopped searching for such a drug, but they have gained little.

Cannflavin A is a flavonoid compound isolated from hemp, and its structural formula[5] is as shown by a formula A below. Cannflavin A is non-addictive. Currently, there are few studies on the pharmacological effects of cannflavin A. It is reported in literatures that cannflavin A participates in microbial metabolism and has antimicrobial properties, anti-inflammatory properties and the like[6].

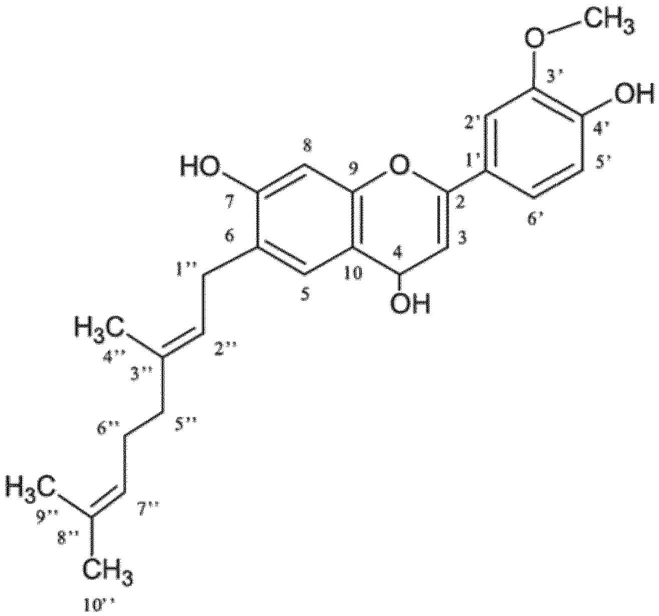

Formula A

ROBLEDO SM ET AL, "Studies In Vitro and In Vivo of antileishmanial activity and Differential Cytotoxicity of Cannabis spp.", 65TH INTERNATIONAL CONGRESS AND ANNUAL MEETING OF THE SOCIETY FOR MEDICINAL PLANT AND NATURAL PRODUCT RESEARCH (GA 2017), vol. 4, disclosed the scientific findings on the potential of Cannabis to treat skin lesions, discovering the role played by the endocannabinoid system in maintaining healthy skin and wound healing, and validating the potential of Cannabis in the topical treatment of cutaneous lesions.

FRANKOVA A ET AL, "In vitro Antimicrobial and Antioxidant Activity of Extracts from Six Chemotypes of Medicinal Cannabis", PLANTA MEDICA, vol. 81, pages S 1 - S381, abstract, evaluated antimicrobial and antioxidant ability of extracts from high potent Cannabis sativa chemotypes.

The diabetic patients are vulnerable to trauma and difficult to recover because their own body protection abilities are weakened and their nerve sensitivity is low. Therefore, it is urgent to develop a novel drug capable of effectively treating trauma.

## SUMMARY

This study explores the efficacy of Cannflavin Aby constructing a skin wound infection model of type II diabetes. It is found that the wound healing of mice in a group subjected to continuous administration is relatively good and is obviously better than that of control groups administrated with soybean isoflavones and quercetin, and is close to the recovery condition of a normal control group. Therefore, it is found that cannflavin A can effectively promote wound healing, and especially can be applied for prevention and treatment of diabetic feet. Thus, the following invention is provided:
The present invention relates to

(1) Cannflavin A or a pharmaceutically-acceptable salt thereof for use in promoting wound healing, treating or preventing diabetic feet; and
(2) a combined pharmaceutical product for use in promoting wound healing, treating or preventing diabetic feet comprising a first product and a second product that are packaged independently, wherein, the first product is cannflavin A or a pharmaceutically-acceptable salt thereof for use according to (1); the second product is a drug for use in inhibiting bacteria or fungi; wherein the drug for inhibiting bacteria or fungi is one or more selected from silver sulfonamide powder, an erythromycin ointment, gentian violet and Deville medical sterilization liquid dressing.

The references to use for treatment or methods of treatment in the subsequent paragraphs are to be interpreted as references to the compounds, pharmaceutical compositions and medicaments of the present invention for use in a method for the treatment of the human body by therapy.

The pharmaceutically-acceptable salt of cannflavin A includes, but is not limited to, organic ammonium salts, alkali metal salts (sodium salts, potassium salts), alkaline earth metal salts (magnesium salts, strontium salts, calcium

salts), etc.

In some embodiments of the present invention, the pharmaceutically-acceptable salt of cannflavin A may be a salt formed from cannflavin A (CBD) with sodium hydroxide, potassium hydroxide, calcium hydroxide, magnesium hydroxide, aluminum hydroxide, lithium hydroxide, zinc hydroxide, barium hydroxide, ammonia, methylamine, dimethylamine, diethylamine, methylpyridine, ethanolamine, diethanolamine, triethanolamine, ethylenediamine, lysine, arginine, ornithine, choline, N,N'-diphenylmethyl ethylenediamine, chloroprocaine, diethanolamine, procaine, N-benzyl phenylethylamine, N-methylglucosamine, piperazine, tris(hydroxymethyl)-aminomethane, etc.

In some embodiments of the present invention, the pharmaceutically-acceptable ester of cannflavin A may be a monoester formed by cannflavin A and a $C_0$-$C_6$ alkyl carboxylic acid, or a diester formed by cannflavin A and two same or different $C_0$-$C_6$ alkyl carboxylic acids, wherein the $C_0$-$C_6$ alkyl carboxylic acid can be a linear alkyl carboxylic acid, a branched alkyl carboxylic acid, or a cycloalkyl carboxylic acid, such as $HCOOH$, $CH_3COOH$, $CH_3CH_2COOH$, $CH_3(CH_2)_2COOH$, $CH_3(CH_2)_3COOH$, $CH_3(CH_2)_4COOH$, $(CH_3)_2CHCOOH$, $(CH_3)_3CCOOH$, $(CH_3)_2CHCH_2COOH$, $(CH_3)_2CH(CH_2)_2COOH$, $(CH_3)_2CH(CH_3)CHCOOH$, $(CH_3)_3CCH_2COOH$, $CH_3CH_2(CH_3)_2CCOOH$, cyclopropane carboxylic acid, cyclobutane carboxylic acid, and cyclopentane carboxylic acid.

Also disclosed but not claimed are the plant extract containing cannflavin A or the pharmaceutical composition for use in promoting wound healing, treating or preventing diabetic feet.

Also disclosed but not claimed is a pharmaceutical composition, including an effective amount of cannflavin A or a pharmaceutically-acceptable salt or ester thereof, and one or more pharmaceutically-acceptable adjuvants, and further including an effective amount of one or more selected from the following components: a component having the effect of drawing out pus and toxin or eliminating necrotic tissues and promoting granulation, and a component for inhibiting bacteria or fungi.

The preparation forms of the pharmaceutical composition can be any pharmaceutically acceptable dosage forms, and the dosage forms include tablets, sugar-coated tablets, film-coated tablets, enteric-coated tablets, capsules, hard capsules, soft capsules, oral liquids, buccal agents, granules, dissolved medicines, pills, pulvis, pastes, sublimed preparations, suspensions, powders, solutions, injections, suppositories, ointments, plasters, creams, sprays, drops and patches; and preferably oral dosage forms, such as capsules, tablets, oral liquids, granules, pills, pulvis, sublimed preparations, pastes, etc. The oral dosage form may contain common excipients, such as a binder, a filler, a diluent, a tableting agent, a lubricant, a disintegrant, a colorant, a flavoring agent and a humectant, and the tablet may be coated if necessary. Suitable fillers include cellulose, mannitol, lactose and other similar fillers; suitable disintegrants include starch, polyvinylpyrrolidone, and starch derivatives such as sodium starch glycolate; and suitable lubricants include, for example, magnesium stearate. Suitable pharmaceutically acceptable humectants include sodium dodecyl sulfate.

Also disclosed but not claimed is the pharmaceutical composition for use in promoting wound healing, treating or preventing diabetic feet, or promoting fibroblast proliferation.

A further aspect of the present invention relates to a combined pharmaceutical product for use in promoting wound healing, treating or preventing diabetic feet, including a first product and a second product that are packaged independently, wherein, the first product is cannflavin A or a pharmaceutically-acceptable salt or ester thereof; and the second product is a drug for use in inhibiting bacteria or fungi; wherein the drug for inhibiting bacteria or fungi is one or more selected from silver sulfonamide powder, an erythromycin ointment, gentian violet and Deville medical sterilization liquid dressing.

In one or more embodiments of the present invention, for the combined pharmaceutical product, wherein in the item (2), the weight percentage content of cannflavin A is 0.1%-90%, 1%-80%, 5%-50%, 0.1%-10%, 0.1%-5%, 1%-10%, or 1%-5%.

The present invention comprises, for the combined pharmaceutical product, wherein the drug for inhibiting bacteria or fungi is one or more selected from silver sulfonamide powder, an erythromycin ointment, gentian violet and Deville medical sterilization liquid dressing.

The present invention comprises, the combined pharmaceutical product for use in promoting wound healing, treating or preventing diabetic feet.

It should be pointed out that, the use dosage and use method of the active ingredient Cannflavin A and a pharmaceutically-acceptable salt or ester thereof depend on many factors, including the age, body weight, gender, natural health status, nutritional status of the patient, the activity intensity of the compound, the administration time, the metabolic rate, the severity of the disease and the subjective judgment of the physician. Preferably, the dosage of the cannflavin A or the pharmaceutically-acceptable salt or ester thereof used is between 0.1-750 mg/kg body weight/day, more preferably 7.5 mg/kg-750 mg/kg body weight/day, 50 mg/kg-500 mg/kg body weight/day, 50 mg/kg-200 mg/kg body weight/day, 75 mg/kg-500mg/kg body weight/day or 75 mg/kg-200 mg/kg body weight/day, further preferably 50 mg/kg-150 mg/kg body weight/day, and in particular preferably 75 mg/kg-150 mg/kg body weight/day.

In the present invention:

The term "ulcerative wound" refers to localized defects of dermis or subcutaneous tissues caused by factors of infection, circulatory disturbance, tumor necrosis, trauma and the like. For example, if a wound does not heal after long term of

treatment, then the growing granulation tissue ages, and the surface of the wound will stop shrinking, which can form refractory ulcers. The ulcer is a localized defect in deep dermis or subcutaneous tissues of skin or a mucous membrane, and its morphology, size and depth may vary depending on the cause of disease and the severity of illness. A ulcer surface often has serous fluid, pus fluid, blood or necrotic tissues. In one embodiment of the present invention, the ulcerative wound is diabetic feet.

The term "non-ulcerative wound" or "non-ulcer wound" refers to common injuries of skin and soft tissues. The "non-ulcerative wound" or "non-ulcer wound" can heal quickly as long as the wound is treated correctly without infection.

The term "effective amount" refers to a dose that can achieve the treatment, prevention, alleviation and/or relief of the disease or condition of the present invention in a subject. The term "subject" may refer to a patient or other animals, especially mammals, such as human, a dog, a monkey, a cow, a horse, etc., which receive cannflavin A to treat, prevent, alleviate, and/or relieve the disease or condition of the present invention.

The term "disease and/or condition" refers to a physical state of the subject that is related to the disease and/or condition of the present invention.

In the present invention, unless otherwise specified, the first product and the second product are only for distinction in reference or clear description, and do not have the meaning of order.

Beneficial effects of the present invention

The cannflavin A or the pharmaceutically-acceptable salt or ester thereof can effectively promote wound healing, and has the potential of preventing and treating diabetic feet.

## BRIEF DESCRIPTION OF THE DRAWINGS

Fig. 1A shows initial blood glucose of mice in a fasting state before establishment of a diabetic model, wherein control represents a control group, and STZ represents an experimental group (mice of a type II diabetics model).

Fig. 1B shows blood glucose of mice in a fasting state after establishment of the diabetic model, wherein control represents a control group, and STZ represents an experimental group (mice of a type II diabetics model).

Fig. 1C shows the body weight of mice in a fasting state before establishment of the diabetic model, wherein control represents a control group, and STZ represents an experimental group (mice of a type II diabetics model).

Fig. 1D shows the body weight of mice in a fasting state after establishment of the diabetic model, wherein control represents a control group, and STZ represents an experimental group (mice of a type II diabetics model).

Fig. 2A-1 shows a wound healing condition of mice in a normal control group on day 0.

Fig. 2A-2 shows a wound healing condition of mice in a normal control group on day 4.

Fig. 2A-3 shows a wound healing condition of mice in a normal control group on day 7.

Fig. 2B-1 shows a wound healing condition of mice in a group administrated with vaseline on day 0 of administration.

Fig. 2B-2 shows a wound healing condition of mice in a group administrated with vaseline on day 4 of administration.

Fig. 2B-3 shows a wound healing condition of mice in a group administrated with vaseline on day 7 of administration.

Fig. 2C-1 shows a wound healing condition of mice in a group administrated with a drug A on day 0 of administration.

Fig. 2C-2 shows a wound healing condition of mice in a group administrated with a drug A on day 4 of administration.

Fig. 2C-3 shows a wound healing condition of mice in a group administrated with a drug A on day 7 of administration.

Fig. 2D-1 shows a wound healing condition of mice in a group administrated with a drug B on day 0 of administration.

Fig. 2D-2 shows a wound healing condition of mice in a group administrated with a drug B on day 4 of administration.

Fig. 2D-3 shows a wound healing condition of mice in a group administrated with a drug B on day 7 of administration.

Fig. 2E-1 shows a wound healing condition of mice in a group administrated with cannflavin A on day 0 of administration.

Fig. 2E-2 shows a wound healing condition of mice in a group administrated with cannflavin A on day 4 of administration.

Fig. 2E-3 shows a wound healing condition of mice in a group administrated with cannflavin A on day 7 of administration.

From Figs. 2A-1 to 2E-3 above, one representative mouse is selected from each group to display the wound healing condition on days 0, 4 and 7.

Fig. 3 shows a wound healing degree of mice in each group on days 0, 4 and 7 of administration.

Fig. 4A shows a colony growth condition of the control group.

Fig. 4B shows a colony growth condition of the group administrated with cannflavin A, wherein, the added cannflavin A is partially precipitated, and the final concentration reference range is 0.05-0.1 mg/ml.

Fig. 5A shows a HE staining result of a normal skin wound tissue.

Fig. 5B shows a HE staining result of a skin wound tissue infected with Staphylococcus aureus, wherein a position indicate by an arrow is a position where inflammatory cell infiltration exists.

Fig. 6A-1 shows a wound healing condition of mice in a normal control group on day 0.

Fig. 6A-2 shows a wound healing condition of mice in a normal control group on day 4.

Fig. 6A-3 shows a wound healing condition of mice in a normal control group on day 7.

Fig. 6B-1 shows a wound healing condition of mice in a group administrated with vaseline on day 0 of administration.

Fig. 6B-2 shows a wound healing condition of mice in a group administrated with vaseline on day 4 of administration.

Fig. 6B-3 shows a wound healing condition of mice in a group administrated with vaseline on day 7 of administration.

Fig. 6C-1 shows a wound healing condition of mice in an experiment group 1 on day 0 of administration.

Fig. 6C-2 shows a wound healing condition of mice in an experiment group 1 on day 4 of administration.

Fig. 6C-3 shows a wound healing condition of mice in an experiment group 1 on day 7 of administration.

Fig. 6D-1 shows a wound healing condition of mice in an experiment group 2 on day 0 of administration.

Fig. 6D-2 shows a wound healing condition of mice in an experiment group 2 on day 4 of administration.

Fig. 6D-3 shows a wound healing condition of mice in an experiment group 2 on day 7 of administration.

Fig. 6E-1 shows a wound healing condition of mice in an experiment group 3 on day 0 of administration.

Fig. 6E-2 shows a wound healing condition of mice in an experiment group 3 on day 4 of administration.

Fig. 6E-3 shows a wound healing condition of mice in an experiment group 3 on day 7 of administration.

From Figs. 6A-1 to 6E-3 above, one representative mouse is selected from each group to display the wound healing condition on days 0, 4 and 7.

Fig. 7 shows the wound healing degree of mice in each group on days 0, 4 and 7 of administration.

Notes: Lesion size refers to the size of the wound, and the size of the wound constructed on the first day is regarded as 100%. Due to the condition of free movement of mice in the later healing process, the wound may be aggravated such as cracking, and the wound healing degree greater than 100% is normal.

Fig. 8A-1 shows a wound healing condition of mice in a normal control group on day 0.

Fig. 8A-2 shows a wound healing condition of mice in a normal control group on day 2.

Fig. 8A-3 shows a wound healing condition of mice in a normal control group on day 4.

Fig. 8A-4 shows a wound healing condition of mice in a normal control group on day 8.

Fig. 8A-5 shows a wound healing condition of mice in a normal control group on day 10.

Fig. 8A-6 shows a wound healing condition of mice in a normal control group on day 14.

Fig. 8B-1 shows a wound healing condition of mice in a type II diabetes control group administrated with vaseline on day 0 of administration.

Fig. 8B-2 shows a wound healing condition of mice in a type II diabetes control group administrated with vaseline on day 2 of administration.

Fig. 8B-3 shows a wound healing condition of mice in a type II diabetes control group administrated with vaseline on day 4 of administration.

Fig. 8B-4 shows a wound healing condition of mice in a type II diabetes control group administrated with vaseline on day 8 of administration.

Fig. 8B-5 shows a wound healing condition of mice in a type II diabetes control group administrated with vaseline on day 10 of administration.

Fig. 8B-6 shows a wound healing condition of mice in a type II diabetes control group administrated with vaseline on day 14 of administration.

Fig. 8C-1 shows a wound healing condition of mice in a type II diabetes group administrated with vaseline + a drug A on day 0 of administration.

Fig. 8C-2 shows a wound healing condition of mice in a type II diabetes group administrated with vaseline + a drug A on day 2 of administration.

Fig. 8C-3 shows a wound healing condition of mice in a type II diabetes group administrated with vaseline + a drug A on day 4 of administration.

Fig. 8C-4 shows a wound healing condition of mice in a type II diabetes group administrated with vaseline + a drug A on day 8 of administration.

Fig. 8C-5 shows a wound healing condition of mice in a type II diabetes group administrated with vaseline + a drug A on day 10 of administration.

Fig. 8C-6 shows a wound healing condition of mice in a type II diabetes group administrated with vaseline + a drug A on day 14 of administration.

Fig. 8D-1 shows a wound healing condition of mice in a type II diabetes group administrated with vaseline + a drug B on day 0 of administration.

Fig. 8D-2 shows a wound healing condition of mice in a type II diabetes group administrated with vaseline + a drug B on day 2 of administration.

Fig. 8D-3 shows a wound healing condition of mice in a type II diabetes group administrated with vaseline + a drug

B on day 4 of administration.

Fig. 8D-4 shows a wound healing condition of mice in a type II diabetes group administrated with vaseline + a drug B on day 8 of administration.

Fig. 8D-5 shows a wound healing condition of mice in a type II diabetes group administrated with vaseline + a drug B on day 10 of administration.

Fig. 8D-6 shows a wound healing condition of mice in a type II diabetes group administrated with vaseline + a drug B on day 14 of administration.

Fig. 8E-1 shows a wound healing condition of mice in a type II diabetes group administrated with vaseline + cannflavin A on day 0 of administration.

Fig. 8E-2 shows a wound healing condition of mice in a type II diabetes group administrated with vaseline + cannflavin A on day 2 of administration.

Fig. 8E-3 shows a wound healing condition of mice in a type II diabetes group administrated with vaseline + cannflavin A on day 4 of administration.

Fig. 8E-4 shows a wound healing condition of mice in a type II diabetes group administrated with vaseline + cannflavin A on day 8 of administration.

Fig. 8E-5 shows a wound healing condition of mice in a type II diabetes group administrated with vaseline + cannflavin A on day 10 of administration.

Fig. 8E-6 shows a wound healing condition of mice in a type II diabetes group administrated with vaseline + cannflavin A on day 14 of administration.

From Figs. 8A-1 to 8E-6 above, one representative mouse is selected from each group to display the wound healing condition on days 0, 2, 4, 8, 10 and 14.

Fig. 9 shows a wound healing degree of mice in each group on days 0-14 of administration.

Notes: Lesion size refers to the size of the wound, and the size of the wound constructed on the first day is regarded as 100%. Due to the condition of free movement of mice in the later healing process, the wound may be aggravated such as cracking, and the wound healing degree greater than 100% is normal.

Fig. 10 shows a curve of cell proliferation after treatment with different concentrations of cannflavin A. * indicates $p < 0.1$, and ** indicates $p < 0.01$.

## DETAILED DESCRIPTION

Hereinafter, embodiments of the present invention will be described in detail with reference to examples, but those skilled in the art will understand that the following examples are only for illustrating the present invention. If no specific conditions are specified in the examples, the examples will be carried out according to conventional conditions or the conditions recommended by the manufacturer. All of the used reagents or instruments which are not specified with the manufacturer are conventional commercially-available products.

Example 1: establishment of type II diabetes model

The method of establishing a type II diabetes model by using low dose of streptozotocin in combination with a high-fat feed[7] is a relatively mature method at present, and has the advantages of short time consumption, high success rate of model establishment, good repeatability, low mortality, and etc.

1. Experimental animals and grouping

25 6-8-week-old male mice (C57BL/6, purchased from Vital River), with a body weight of 20 g or above, were used.

A control group (control): 5 mice, intraperitoneally injected with 0.1 mmol/L of a citrate buffer solution with a pH of 4.2.

An experimental group (STZ): 50 mice, intraperitoneally injected with 1% streptozotocin; 25 mice were randomly selected for statistics.

2. Method for model establishment

Before model establishment, the initial blood glucose and body weight of mice in a fasting state were respectively determined, as shown in Figs. 1A and 1C respectively. The mice in a fasting state were intraperitoneally injected with 1% streptozotocin (50 mg/kg). The streptozotocin was prepared with 0.1 mmol/L of a citrate buffer solution with a pH of 4.2, and was prepared immediately before use. The mice were given high-fat feed 1 hour after the injection, and the state of the mice were observed closely. Insulin or glucose was supplemented in time according to the state of the mice

(generally, when blood glucose was too low, cachexia, weak and limp, severe breathing weakness and even death would appear, and if such a situation occurred, glucose should be supplemented in time; and when blood glucose is too high, mice would have frequent micturition and dizziness, which can be determined by detecting blood glucose, and in severe cases, insulin should be properly supplemented) for 5 consecutive days.

On day 6 of model establishment, the blood glucose of the mice in a fasting state was detected, and the mice that did not reach 199.8 mg/dl were supplemented with streptozotocin once, and then the fasting blood glucose was detected. When the blood glucose of the mice was stable to be 199.8 mg/dl or above, the model was established successfully.

3. Experimental results

The results were respectively shown in Figs. 1B and 1D.

The results showed that the blood glucose and body weight after the establishment of the type II diabetes model through induction decreased continuously, the blood glucose was greater than 199.8 mg/dl, and the mice had diabetic symptoms such as drinking more water and excreting more, and thus the model was successfully established.

Example 2: Experiment of skin wound healing

1. Experimental method

(1) Establishment of skin wound model

Wild C57 adult male mice (C57BL/6, purchased from Vital River) were taken and anesthetized, and then subjected to backside unhairing to establish a whole cortex wound with a 6 mm biopsy puncher. Attention should be paid to disinfection for preventing infection.

(2) Grouping and mode of administration

A normal control group: 5 mice, no administration.

A group administrated with vaseline: vaseline (vaseline for medical purpose, purchased from Tianjin Zhiyuan Chemical Reagent Co., Ltd., the same below), 5 mg vaseline/mouse, 5 mice.

A group administrated with A drug A: 10 mg/mouse, 10 mice (as a flavonoid control, and the drug A was soybean isoflavones).

A group administrated with a drug B: 10 mg/mouse, 10 mice (as a flavonoid control, and the drug B was quercetin).

A group administrated with cannflavin A: cannflavin A, 10 mg/mouse, 10 mice.

The administration modes of the latter 4 groups were all application onto the wound, and the administration was conducted once a day. Because vaseline was a commonly used ointment base[8], in the latter 3 groups, the drug A, the drug B or cannflavin A were respectively well mixed with 5 mg of vaseline and then administered.

(3) Detection method

Photos were taken to record a wound healing condition of mice on days 0, 4 and 7 of administration, and the healing degree of a wound area was calculated by the PhotoShop software with 1 $cm^2$ as a scale. The specific steps were as follows:

The records of wound healing conditions were all done by taking photos directly. The inventor took photos of a scale of 1 $cm^2$ in the photos as a reference, then sketched the wound area with the PhotoShop software, and then divided the wound area by the scale area to get a relative wound area.

$$\text{Healing degree} = S/S_0 \times 100\%$$

wherein S indicated the existing relative wound area, and $S_0$ indicated the initial relative wound area.

By subtracting the aforementioned healing degree from 1, we could know how much healing had been achieved.

2. Experimental results

The wound healing conditions are shown in Figs. 2A-1 to 2E-3.

The wound healing degrees are shown in Fig. 3.

The results showed that:

(1) the control group, the group administrated with vaseline, the group administrated with the drug A, the group administrated with the drug B and the group administrated with cannflavin A all had good recovery conditions, and the wound basically healed in 4 days, and could completely heal in 7 days.

(2) In terms of promoting the healing degree of wounds without bacterial infection, there was no significant difference among cannflavin A, vaseline, the drug A, the drug B and the control. Without wishing to be limited by theory, for normal and healthy mice, under the condition without infection, the body's self-repair ability was very strong, and small skin wounds would heal by themselves even if they were not given drugs, so it was difficult to fully reflect the effect of the drugs.

Example 3: experiment of healing of skin wound infected with Staphylococcus aureus (1)

Wounds of diabetic patients were not only difficult to heal, but also susceptible to infection. Staphylococcus aureus was the most common pathogen in human pyogenic infection, which could cause not only local pyogenic infection, but also pneumonia, pseudomembranous enteritis, pericarditis, etc., and even systemic infections such as septicemia and sepsis. In this study, the skin infection model of diabetic mice was established by employing Staphylococcus aureus[8], aiming to be closer to a disease in which the human wounds were not easy to heal, and making the research more meaningful.

1. Experimental method

(1) Method for model establishment

normal mice as control and mice with type II diabetes established according to the method of Example 1 were taken, anesthetized, and then subjected to backside unhairing to establish a whole cortex wound with a 6 mm biopsy puncher. The mice were inoculated with $1.5 \times 10^6$ CFU/ml of Staphylococcus aureus.

(2) Method for preparing Staphylococcus aureus

A LB liquid medium was inoculated with Staphylococcus aureus at 1:100 to be incubated at 37°C and 220 rpm to an exponential growth phase. OD 600 was detected. The bacterial solution was diluted to $10^{-5}$ and $10^{-6}$ folds, coated onto a solid antibiotic-free LB culture plate, and cultured overnight at 37°C to calculate the number of colony units.

(3) Grouping and mode of administration

A normal control group: no administration, 5 mice.

A group administrated with vaseline: vaseline, 5 mg vaseline/mouse, 5 mice.

An experiment group 1: cannflavin A, 0.15 mg/mouse, 5 mice.

An experiment group 2: cannflavin A, 1.5mg/mouse, 5 mice.

An experiment group 3: cannflavin A, 15mg/mouse, 5 mice.

The aforementioned 5 groups were all inoculated with Staphylococcus aureus according to the previous step (1).

The administration modes of the latter 4 groups were all application onto the wound, and the administration was conducted once a day. In the latter 3 groups, different doses of cannflavin A were respectively well mixed with 5 mg of vaseline as an ointment base and then administered.

(4) Detection method

Photos were taken to record the wound healing condition of the mice after administration, and the healing degree of a wound area was calculated by the PhotoShop software with 1 $cm^2$ as a scale. The specific method was the same as that of Example 2.

2. Experimental results

(1) Results of inhibition of cannflavin A on Staphylococcus aureus

The results were shown in Figs. 4A and 4B.

When OD600 = 0.85, the colony density was $1.2*10^9$ CFU/ml, and Cannflavin A had an obvious inhibition effect on Staphylococcus aureus.

(2) HE staining results of paraffin sections of the whole cortex wound on day 1 after the inoculation with Staphylococcus aureus

The results were shown in Figs. 5A and 5B. Wherein:

In Fig. 5, the structure of the skin wound tissue of the type II diabetic mice without inoculation with Staphylococcus aureus was complete; and
in Fig. 5B, after the inoculation with Staphylococcus aureus, the structure of the skin wound tissue of the type II diabetic mice was severely damaged, and there was inflammatory cell infiltration.

After inoculation with Staphylococcus aureus, the structure of the skin tissue was seriously damaged, wherein the epidermal layer and stratum spinosum were relatively thicker, the cuticular layer was broken, inflammatory cell infiltration occurred in the corium layer (at the position indicated by the arrow), and meanwhile the hair follicle structure was destroyed.

(3) The wound healing conditions of each group were shown in Figs. 6A-1 to 6E-3. The wound healing degrees were shown in Fig. 7.

The results showed that: when the administration dosage of Cannflavin A was 0.15 mg/mouse, no obvious effect was achieved, and there was no obvious difference compared with the group administrated with vaseline; when the administration dosage was 1.5 mg/mouse, an obvious effect was achieved; and when the administration dosage was 15 mg/mouse, an obvious effect was also achieved, and there was no significant difference compared with that when the dosage was 1.5 mg/mouse.

Example 4: experiment of healing of skin wound infected with Staphylococcus aureus (2)

1. Experimental method

The experimental method was the same as that of Example 3, except that the grouping and administration were as follows:
A normal control group: no administration, 5 mice.

A type II diabetes control group administrated with vaseline: vaseline, 5 mg vaseline/mouse, 5 mice.

A type II diabetes group administrated with vaseline + a drug A: 10 mg/mouse, 10 mice (as a flavonoid control, and the drug A was soybean isoflavones).

A type II diabetes group administrated with vaseline + a drug B: 10 mg/mouse, 10 mice (as a flavonoid control, and the drug B was quercetin).

A type II diabetes group administrated with vaseline + cannflavin A: 1.5 mg/mouse, 10 mice.

The aforementioned 5 groups were all inoculated with Staphylococcus aureus according to the previous step (1).

The administration modes of the latter 4 groups were all application onto the wound, and the administration was conducted once a day. In the latter 3 groups, the drug A, the drug B or cannflavin A were respectively well mixed with 5 mg of vaseline as an ointment base and then administered.

2. Experimental results

The wound healing conditions of each group were shown in Figs. 8A-1 to 8E-6.

The wound healing degrees were shown in Fig. 9.

The results showed that the healing degree of the group administrated with cannflavin A was obviously superior to those of the diabetic control group administrated with vaseline, the group administrated with the drug A and the group administrated with the drug B, and the recovery condition was close to that of the normal control group.

Without wishing to be limited by theory, the drug A and the drug B were both flavonoid compounds, which had common bacteriostatic properties, but the bacteriostatic properties could only relieve symptoms, but could not help wound healing. The ability of cannflavin A in accelerating wound healing should be specific to it.

Furthermore, under normal circumstances, the aseptic wounds of mice basically did not need any drug assistance. As long as the wounds were cleaned and disinfected appropriately, they would not deteriorate and could be cured. However, the mice were at the SPF level, while people's living environment could not be at the SPF level, so it was impossible to avoid bacterial infection. Inoculating the mice with Staphylococcus aureus could better imitate and approach the real situation of human patients.

Example 5: cell experiment of promoting skin wound healing with cannflavin A

Skin wound repair needed to go through several different stages, including a hemostasis phase, an inflammation phase, a proliferation phase and a remodeling phase, wherein the proliferation phase involved the proliferation of fibroblasts, and in the remodeling phase, the fibroblasts were activated by macrophages and differentiated into muscle fibroblasts to participate in the long-term remodeling process. The inventor also studied the effect of cannflavin A on the proliferation ability of the fibroblasts, and further explored the possible mechanism of cannflavin A in promoting wound healing.

1. Experimental materials and instruments

experimental cell line-mouse embryonic fibroblast cell line (NIH-3T3)-purchased from Central Laboratory, Union Medical Hospital on the National Experimental Cell Resource Sharing Platform
fetal bovine serum (FBS)-GIBCO, United States
DMEM basal medium-GIBCO, United States
Trypsin-Sigma-Aldrich, United States
EDTA-Sigma-Aldrich, United States
DMSO-Sigma-Aldrich, United States
MTT cell proliferation and cytotoxicity detection kit-MP, United States
Cell cryopreservation tubes-Corning
Tabletop centrifuge-eppendorf, United States
$CO_2$ incubator-Sanyo, Japan
Electric-heated thermostatic water bath kettle-Shanghai Yiheng Technology Co., Ltd. Multifunctional microwell plate detector-BIOTEK, United States

2. Experimental method

(1) Cell culture

Cell thawing and culture: the cryopreserved cells were taken out from liquid nitrogen, and then shaken continuously in a water bath kettle at 37°C to promote thawing of the cells. The cells were transferred into a 15 ml centrifuge tube, 10 ml of a preheated DMEM complete medium containing 10% FBS was added, and the mixture was well blown gently, and centrifuged at 800 rpm for 5 min, and the supernatant was discarded. 10 ml of a DMEM complete medium containing 10% FBS was added to the cell pellet to be blown gently, a plate of 10 cm was inoculated with the mixture, and the cell pellet was incubated in a cell incubator containing 5% $CO_2$ at 37°C. On the next day, the medium was replaced by a fresh culture medium, and the culture was continued.

Cell passage: when the cell density reached 80%-90%, the culture medium was removed, and the cells were washed with 5 ml PBS twice. 3 ml of 0.25% trypsin was added, and the cells containing the trypsin were put into a cell incubator for incubation for 1-2 min. The states of the cells were observed under a microscope. The digestion solution was removed by sucking when the cells became round and the intercellular gaps became larger, a DMEM complete medium containing 10% FBS was added to stop digestion, and the mixture was blown repeatedly with a pipette to be mixed uniformly, and passage was conducted at a certain proportion according to the experimental requirements.

Preparation of 0.25% trypsin: 0.25 g of trypsin and 0.03 g of EDTA were weighed and dissolved in 100 ml of PBS, mixed uniformly, filtered through a 0.22 $\mu$m filter membrane, sterilized, and then stored at 4°C.

Cell cryopreservation: when the cell density reached 80%-90%, the culture medium was removed, and the cells were washed with 5 ml PBS twice. 3 ml of 0.25% trypsin was added, and the cells containing the trypsin were put into a cell incubator for 1-2 min. 5 ml of a DMEM complete medium was added to stop pancreatin digestion, the mixture was transferred into a 15 ml centrifuge tube, and centrifuged at 800 rpm for 5 min, and the supernatant was discarded. The cell pellet was suspended with a cryoprotectant, mixed uniformly, packed into cryopreservation tubes at 1 ml/tube, marked, wrapped with cotton, and then sequentially subjected to be at 4°C for 20 minutes, at -20°C for 2 hours, and at -80°C overnight, and finally put into liquid nitrogen for long-term storage. The preparation of the cryoprotectant: 70% DMEM basal medium + 20% FBS + 10% DMSO, wherein DMSO should be added dropwise slowly and shaken while adding dropwise.

(2) Detection of cell proliferation by methyl thiazolyl tetrazolim (MTT) assay.

Preparation of MTT solution: 0.5 g of MTT was weighed into 100 ml PBS to be prepared into a 5 mg/ml MTT solution. It could be used immediately after preparation, or it could be stored directly at -20°C with protection from light,

or it could be subpackaged appropriately as required and then stored at -20°C with protection from light.

According to the experimental requirements, 100 microliters of about 2,000 cells was added into each well of a 96-well plate, and a solvent control group (i.e., a mock group, using a complete medium, no cell addition) and experiment groups of 0 μM cannflavin A, 5 μM cannflavin A, 10 μM cannflavin A and 20 μM cannflavin A were respectively set, and 8 replicate wells were set. The cells were incubated in a cell incubator containing 5% $CO_2$ at 37°C for 48 hours.

20 microliters of the MTT solution was added into each well, and the cells were continually incubated in the cell incubator for 4 hours.

150 microliters of DMSO was added into each well, and then the cells were continually incubated in the cell incubator, which could be properly placed in a shaker to facilitate dissolving. The incubation was ended until it was found that the blue crystals were completely dissolved when observed under a common optical microscope. The OD 490 nm was detected on a multifunctional microwell plate detector. The higher OD 490 nm reflected the stronger cell proliferation.

3. Experimental results

The results were shown in Fig. 10.

The results showed that:

The cell proliferation ability of the solvent control group in which the complete medium was used and no cannflavin A was added, was significantly lower than those of the group in which 5 μM cannflavin A was added and the group in which 10 μM cannflavin A was added;

when cannflavin A reached 20 μM, the effect of cannflavin A in promoting NIH-3T3 proliferation was basically the same as that when cannflavin A was 5 μM. It was thus speculated that 10 μM could be used as the optimum concentration.

Therefore, the inventor speculated that the effect of Cannflavin A in promoting skin wound recovery of mice was related to its promotion of the fibroblast proliferation.

Cited References:

1. Basu, A. and D.K. Nandy, Diabetes. 2017.
2. Corbin, K.D., et al., Obesity in Type 1 Diabetes: Pathophysiology, Clinical Impact and Mechanisms. Endocr. Rev., 2018.
3. Castriota, F., et al., Obesity and increased susceptibility to arsenic-related type 2 diabetes in Northern Chile. Environ. Res., 2018. 167: p. 248-254.
4. Veerasubramanian, P.K., et al., Corrigendum to "An investigation of konjac glucomannan-keratin hydrogel scaffold loaded with Avena sativa extracts for diabetic wound healing" [Colloids Surf. B Biointerfaces 165 (2018) 92-102*].* Colloids and Surfaces B: Biointerfaces, 2018. 171: p. 319.
5. Ahmed, S.A., et al., Structure determination and absolute configuration of cannabichromanone derivatives from high potency Cannabis sativa. Tetrahedron letters, 2008. 49(42): p. 6050-6053.
6. Ibrahim, A.K., et al., Microbial metabolism of cannflavin A and B isolated from Cannabis sativa. Phytochemistry, 2010. 71(8-9): p. 1014-9.
7. Ibrahim, A.K., et al., Microbial metabolism of cannflavin A and B isolated from Cannabis sativa. Phytochemistry, 2010. 71(8): p. 1014-1019.
8. de Moura, F.B.R., et al., Pro-Fibrogenic and Anti-Inflammatory Potential of a Polyphenol-Enriched Fraction from Annona crassiflora in Skin Repair. Planta Med, 2018.

## Claims

**1.** Cannflavin A or a pharmaceutically-acceptable salt thereof for use in promoting wound healing, treating or preventing diabetic feet.

**2.** The use according to claim 1, wherein the wound is a non-ulcerative wound or an ulcerative wound.

**3.** A combined pharmaceutical product for use in promoting wound healing, treating or preventing diabetic feet comprising a first product and a second product that are packaged independently,

wherein,

the first product is cannflavin A or a pharmaceutically-acceptable salt thereof for use according to claim 1;
the second product is a drug for use in inhibiting bacteria or fungi;

wherein the drug for inhibiting bacteria or fungi is one or more selected from silver sulfonamide powder, an erythromycin ointment, gentian violet and Deville medical sterilization liquid dressing.

## Patentansprüche

1. Cannflavin A oder ein pharmazeutisch akzeptables Salz davon zur Verwendung bei der Förderung der Wundheilung, Behandlung oder Vorbeugung von diabetischen Füßen.

2. Verwendung gemäß Anspruch 1, wobei die Wunde eine nicht ulzerierende Wunde oder eine ulzerierende Wunde ist.

3. Ein kombiniertes pharmazeutisches Produkt zur Verwendung bei der Förderung der Wundheilung, Behandlung oder Vorbeugung von diabetischen Füßen, das ein erstes Produkt und ein zweites Produkt beinhaltet, die unabhängig voneinander verpackt sind, wobei

das erste Produkt Cannflavin A oder ein pharmazeutisch akzeptables Salz davon zur Verwendung gemäß Anspruch 1 ist;
das zweite Produkt ein Arzneimittel zur Verwendung bei der Hemmung von Bakterien oder Pilzen ist;
wobei das Arzneimittel zur Hemmung von Bakterien oder Pilzen eines oder mehrere, ausgewählt aus Silbersulfonamidpulver, einer Erythromycin-Salbe, Gentianaviolett und medizinischem flüssigem Deville-Sterilisationsverband, ist.

## Revendications

1. Cannflavine A ou un sel pharmaceutiquement acceptable de celle-ci pour une utilisation dans la favorisation de la cicatrisation de plaies, le traitement ou la prévention des pieds diabétiques.

2. L'utilisation selon la revendication 1, dans laquelle la plaie est une plaie non ulcéreuse ou une plaie ulcéreuse.

3. Un produit pharmaceutique combiné pour une utilisation dans la favorisation de la cicatrisation de plaies, le traitement ou la prévention des pieds diabétiques comprenant un premier produit et un deuxième produit qui sont conditionnés indépendamment, dans lequel,

le premier produit est la cannflavine A ou un sel pharmaceutiquement acceptable de celle-ci pour une utilisation selon la revendication 1 ;
le deuxième produit est un médicament pour une utilisation dans l'inhibition de bactéries ou de champignons ;
dans lequel le médicament pour l'inhibition de bactéries ou de champignons est un ou plusieurs médicaments sélectionnés parmi le sulfamide argentique en poudre, un onguent d'érythromycine, du violet de gentiane et un pansement liquide de stérilisation médical Deville.

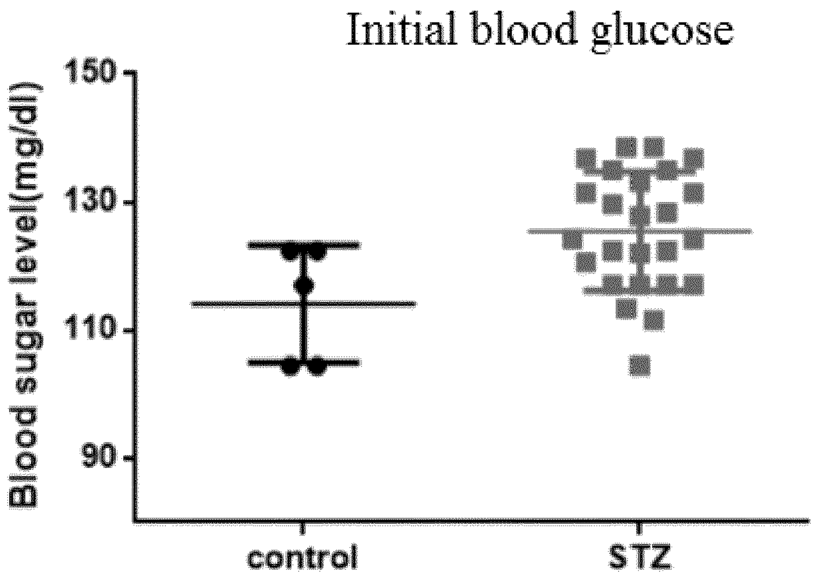
Initial blood glucose
Blood sugar level(mg/dl)
150
130
110
90
control
STZ

Fig. 1A

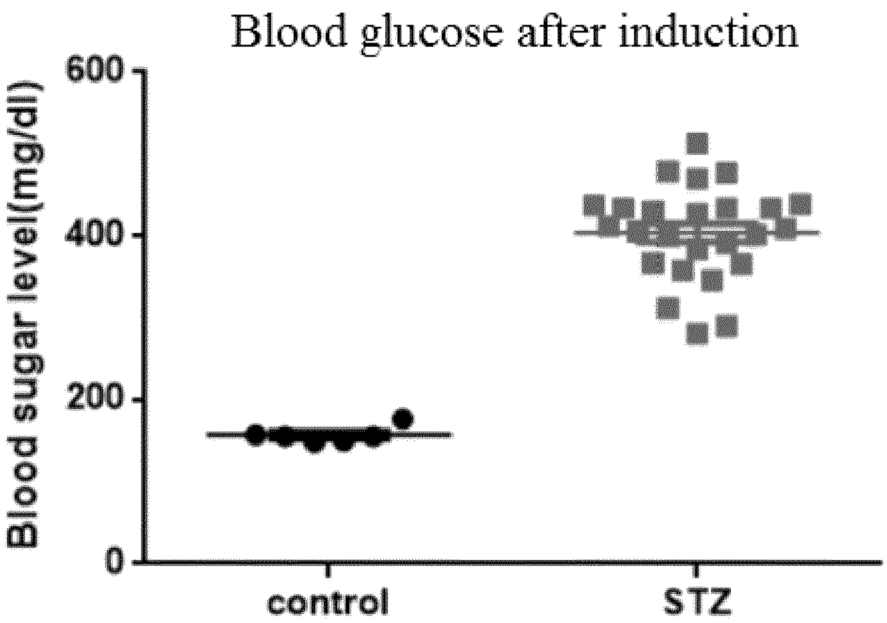
Blood glucose after induction
Blood sugar level(mg/dl)
600
400
200
0
control
STZ

Fig. 1B

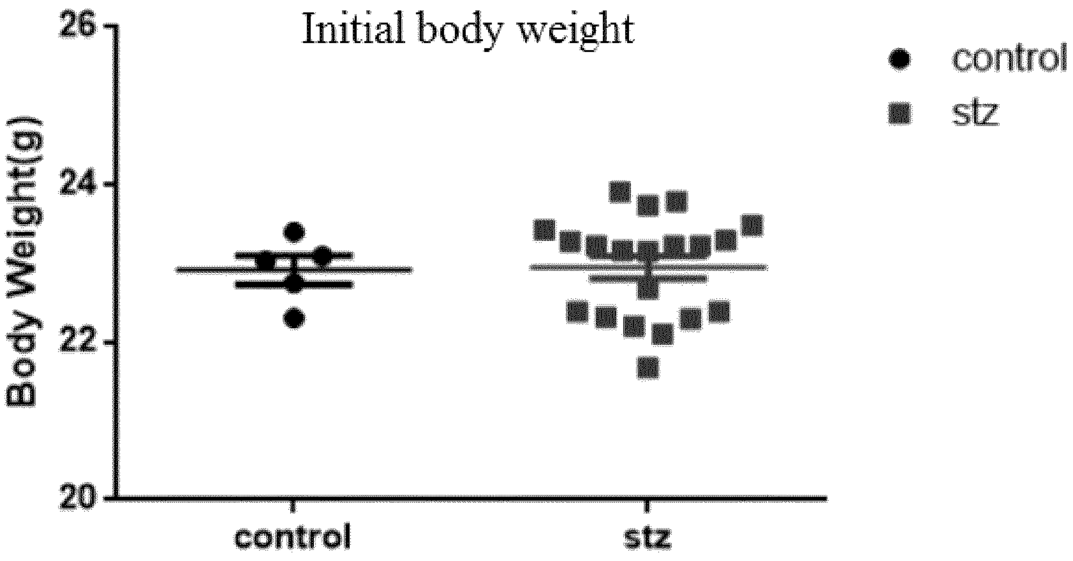


Fig. 1C

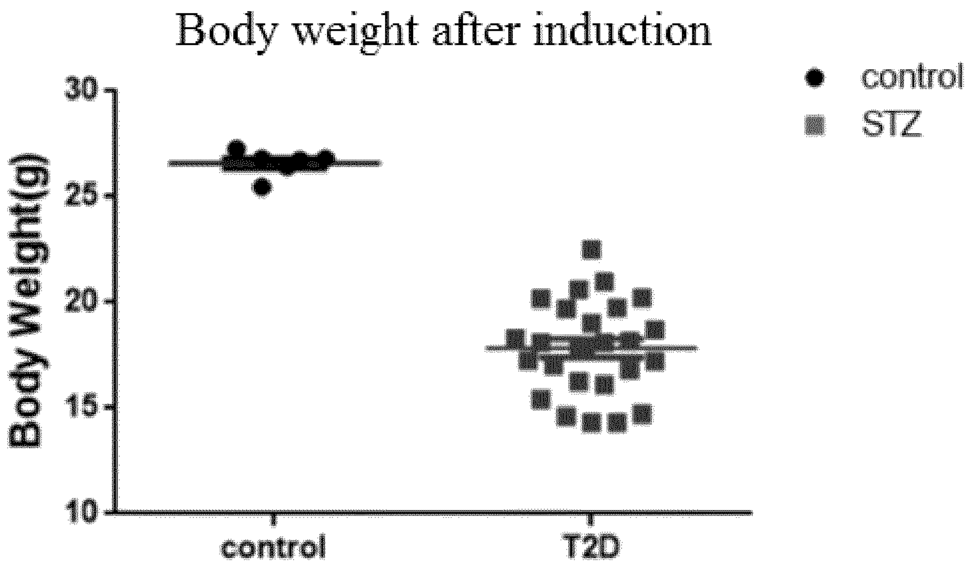


Fig. 1D

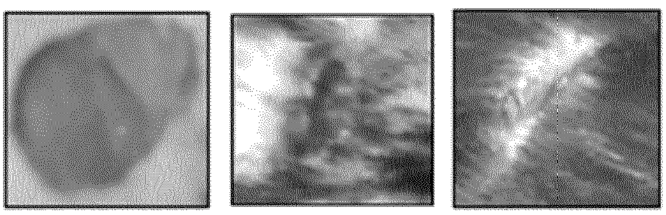

Fig. 2A-1 Fig. 2A-2 Fig. 2A-3

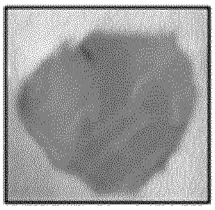

Fig. 2B-1

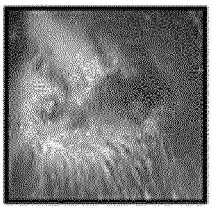

Fig. 2B-2

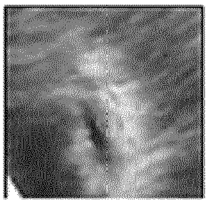

Fig. 2B-3

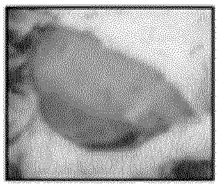

Fig. 2C-1

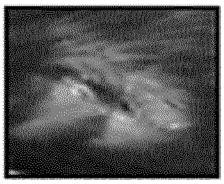

Fig. 2C-2

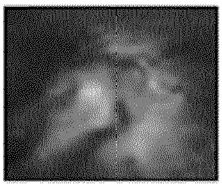

Fig. 2C-3

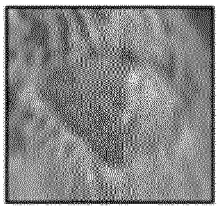

Fig. 2D-1

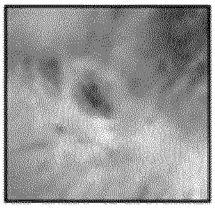

Fig. 2D-2

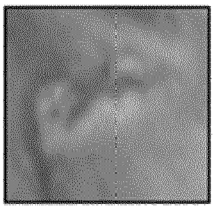

Fig. 2D-3

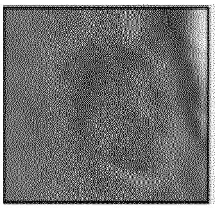

Fig. 2E-1

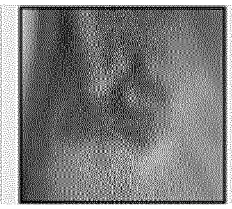

Fig. 2E-2

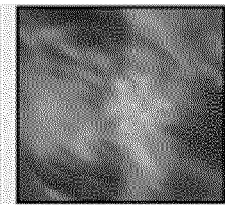

Fig. 2E-3

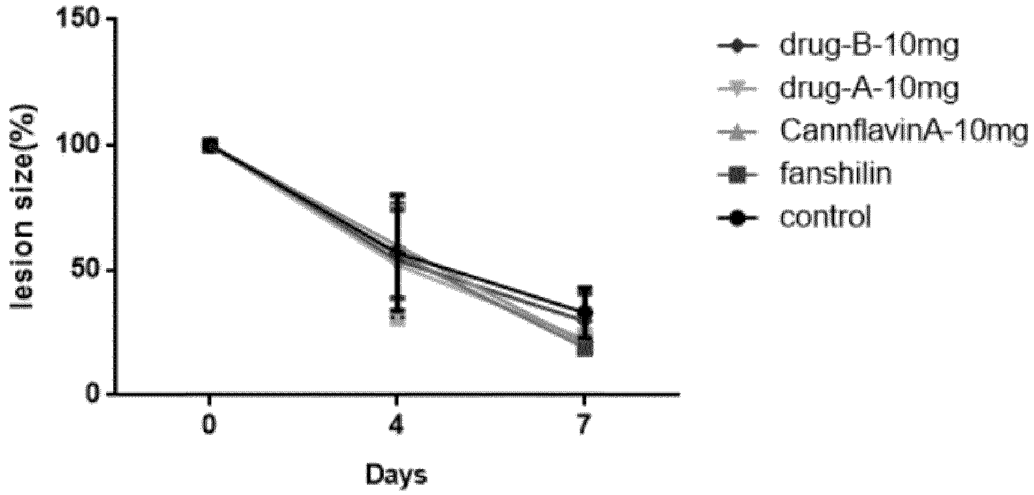


Fig. 3

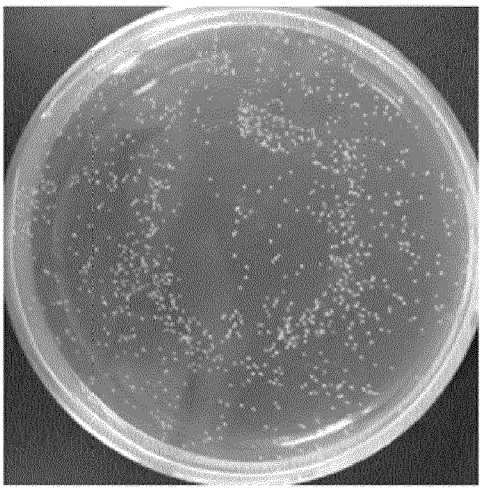

10-5, 1222CFU-100ul

Fig. 4A

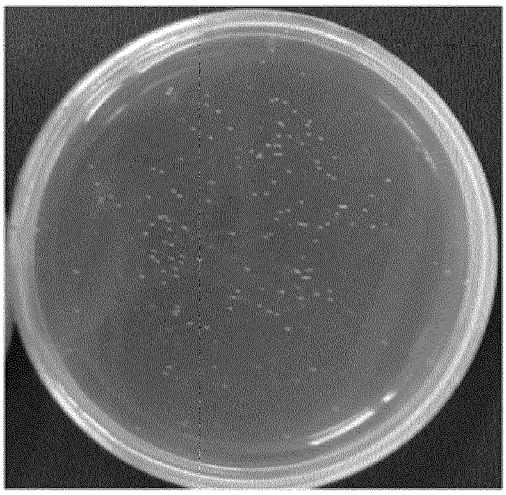

Cannflavin A Treatment

Fig. 4B

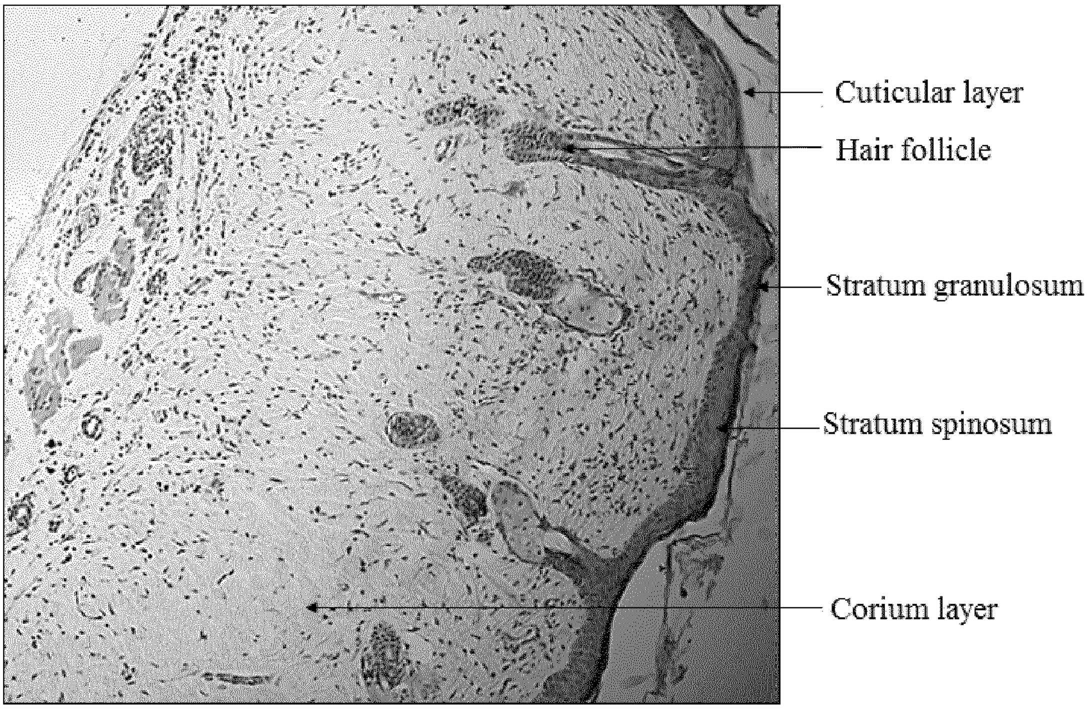
Cuticular layer
Hair follicle
Stratum granulosum
Stratum spinosum
Corium layer

Fig. 5A

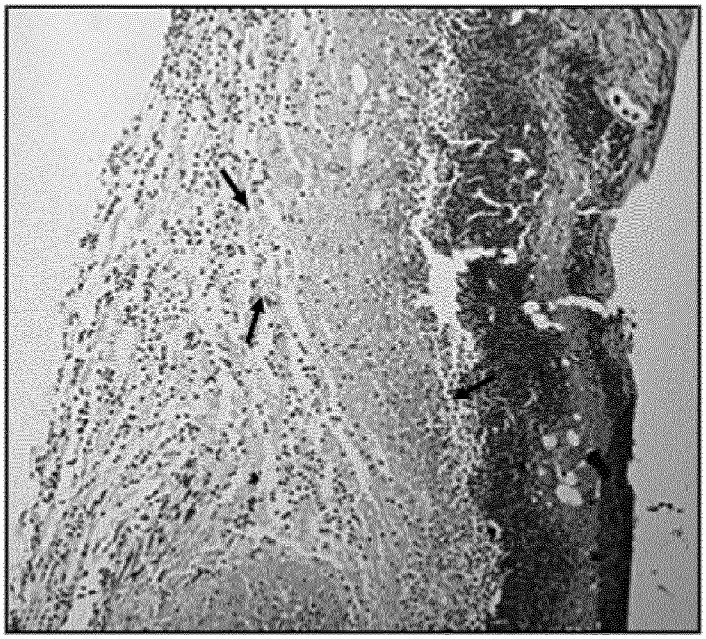

Fig. 5B

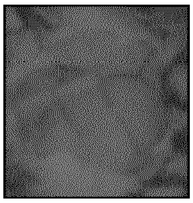
Fig. 6A-1

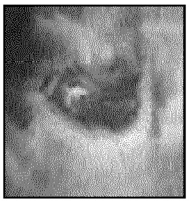
Fig. 6A-2

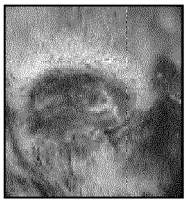
Fig. 6A-3

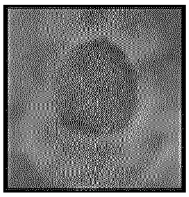
Fig. 6B-1

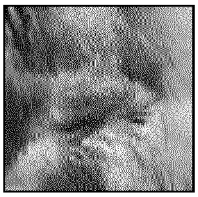
Fig. 6B-2

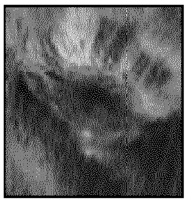
Fig. 6B-3

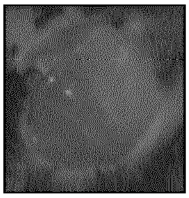
Fig. 6C-1

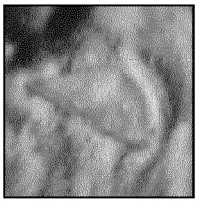
Fig. 6C-2

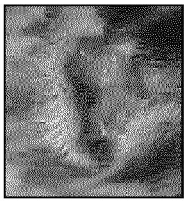
Fig. 6C-3

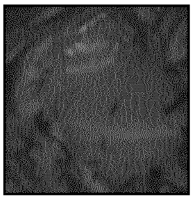
Fig. 6D-1

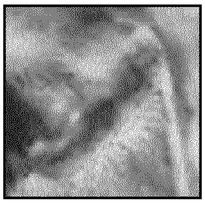
Fig. 6D-2

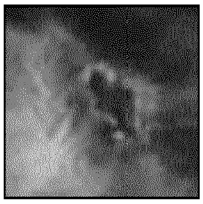
Fig. 6D-3

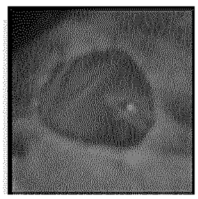
Fig. 6E-1

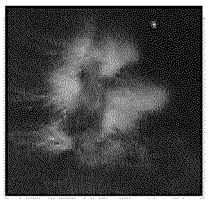
Fig. 6E-2

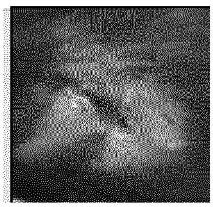
Fig. 6E-3

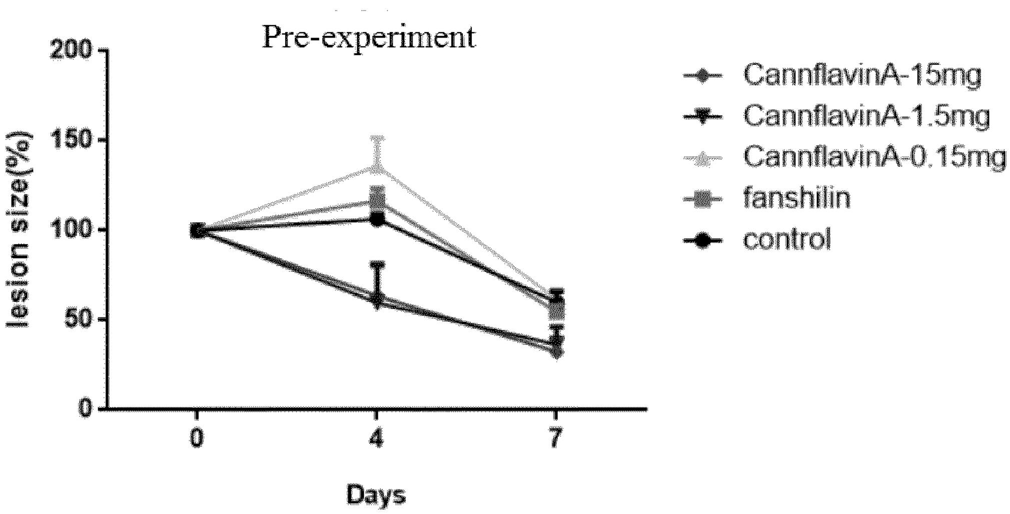


Fig. 7

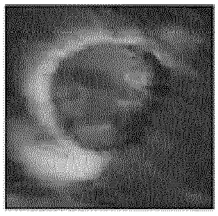

Fig. 8A-1

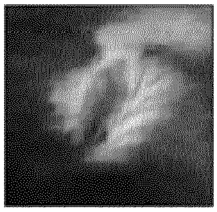

Fig. 8A-2

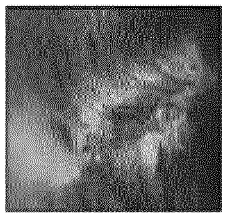

Fig. 8A-3

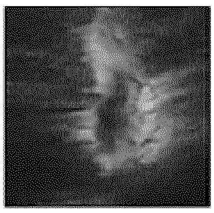

Fig. 8A-4

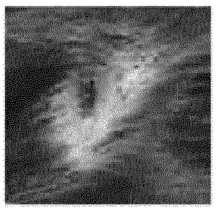

Fig. 8A-5

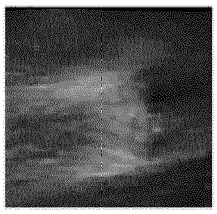

Fig. 8A-6

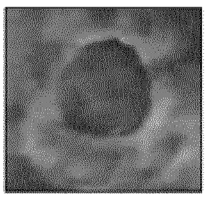

Fig. 8B-1

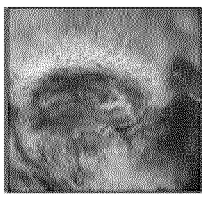

Fig. 8B-2

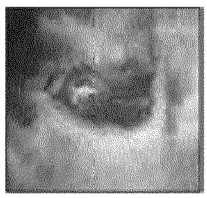

Fig. 8B-3

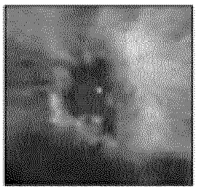

Fig. 8B-4

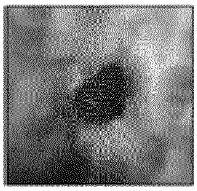

Fig. 8B-5

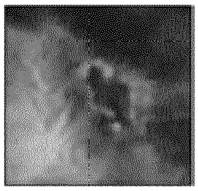

Fig. 8B-6

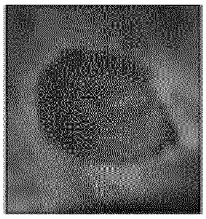

Fig. 8C-1

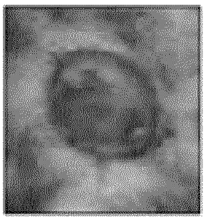

Fig. 8C-2

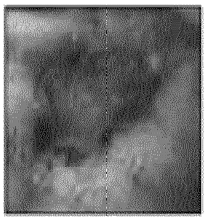

Fig. 8C-3

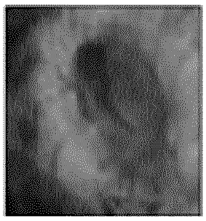

Fig. 8C-4

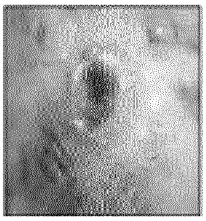

Fig. 8C-5

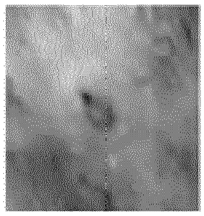

Fig. 8C-6

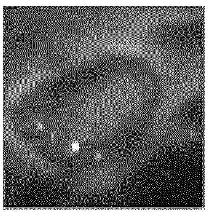

Fig. 8D-1

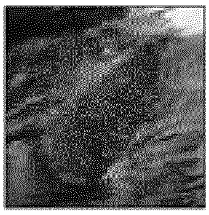

Fig. 8D-2

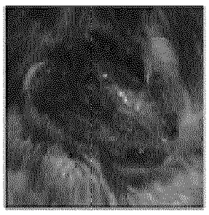

Fig. 8D-3

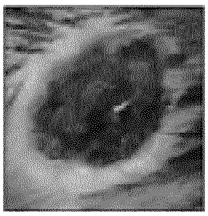

Fig. 8D-4

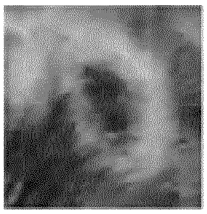

Fig. 8D-5

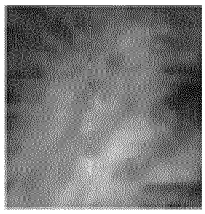

Fig. 8D-6

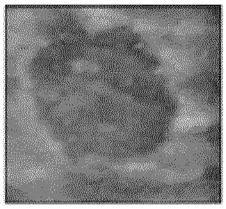

Fig. 8E-1

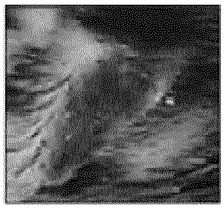

Fig. 8E-2

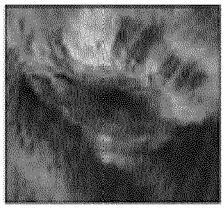

Fig. 8E-3

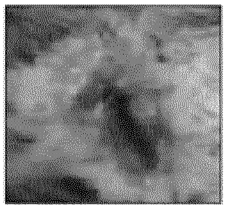

Fig. 8E-4

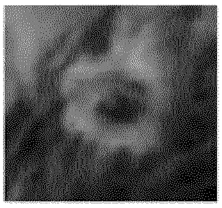

Fig. 8E-5

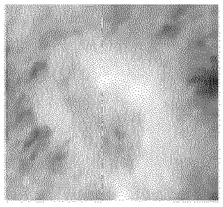

Fig. 8E-6

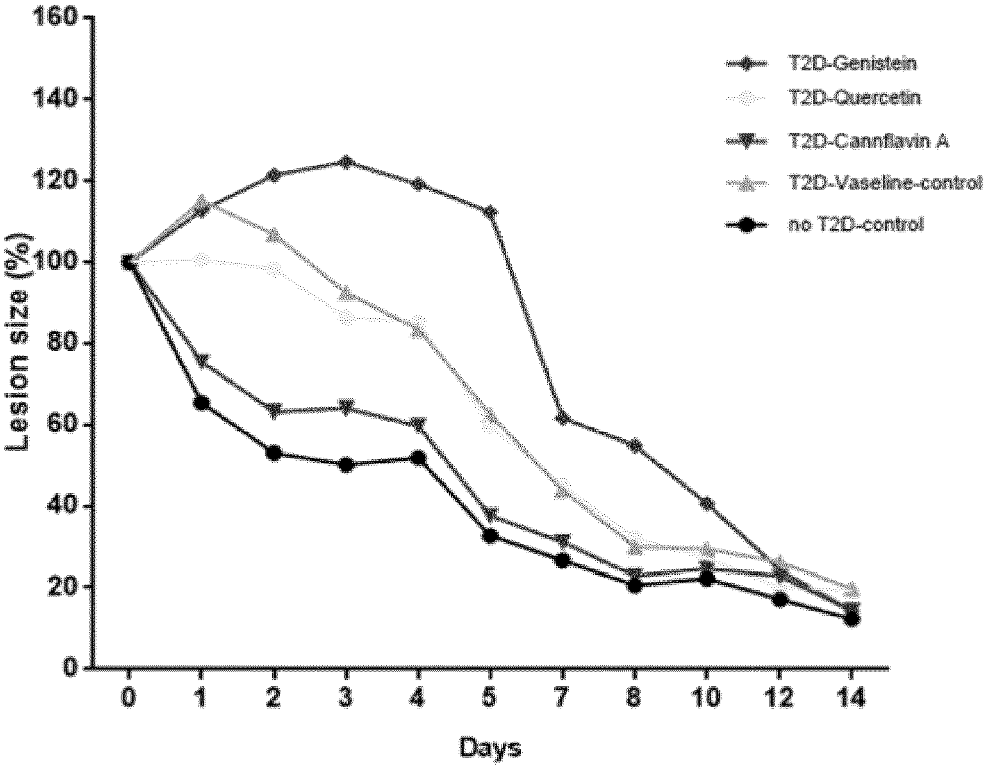


Fig. 9

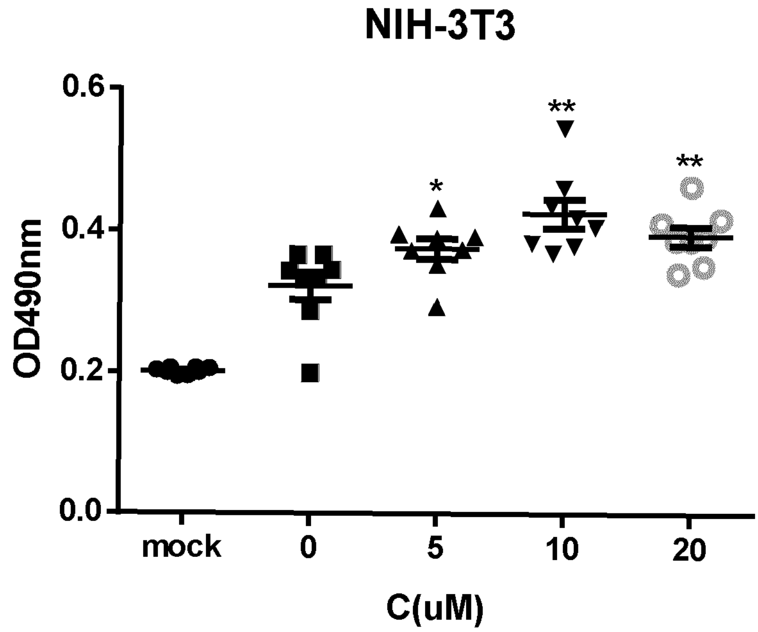


Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ROBLEDO SM et al.** Studies In Vitro and In Vivo of antileishmanial activity and Differential Cytotoxicity of Cannabis spp. *65TH INTERNATIONAL CONGRESS AND ANNUAL MEETING OF THE SOCIETY FOR MEDICINAL PLANT AND NATURAL PRODUCT RESEARCH,* 2017, vol. 4
- **FRANKOVA A et al.** In vitro Antimicrobial and Antioxidant Activity of Extracts from Six Chemotypes of Medicinal Cannabis. *PLANTA MEDICA,* vol. 81, S1-S381
- **BASU, A. ; D.K. NANDY.** *Diabetes,* 2017
- **CORBIN, K.D. et al.** Obesity in Type 1 Diabetes: Pathophysiology, Clinical Impact and Mechanisms. *Endocr. Rev.,* 2018
- **CASTRIOTA, F. et al.** Obesity and increased susceptibility to arsenic-related type 2 diabetes in Northern Chile. *Environ. Res.,* 2018, vol. 167, 248-254
- **VEERASUBRAMANIAN, P.K. et al.** Corrigendum to "An investigation of konjac glucomannan-keratin hydrogel scaffold loaded with Avena sativa extracts for diabetic wound healing. *Colloids Surf. B Biointerfaces,* 2018, vol. 165, 92-102
- *Colloids and Surfaces B: Biointerfaces,* 2018, vol. 171, 319
- **AHMED, S.A. et al.** Structure determination and absolute configuration of cannabichromanone derivatives from high potency Cannabis sativa. *Tetrahedron letters,* 2008, vol. 49 (42), 6050-6053
- **IBRAHIM, A.K. et al.** Microbial metabolism of cannflavin A and B isolated from Cannabis sativa. *Phytochemistry,* 2010, vol. 71 (8-9), 1014-9
- **IBRAHIM, A.K. et al.** Microbial metabolism of cannflavin A and B isolated from Cannabis sativa. *Phytochemistry,* 2010, vol. 71 (8), 1014-1019
- **MOURA, F.B.R. et al.** Pro-Fibrogenic and Anti-Inflammatory Potential of a Polyphenol-Enriched Fraction from Annona crassiflora in Skin Repair. *Planta Med,* 2018